# EUROPEAN PATENT APPLICATION

(11) **EP 4 310 070 A1**
(43) Date of publication of application: **24.01.2024**
(21) Application number: 21930957.2
(22) Date of filing: 31.03.2021
(51) Int. Cl.: C07C 211/52, C07C 215/44, C07C 249/02, C07C 251/24, C07C 209/52, C07C 213/02, A61K 31/137, A61P 11/00, A61P 11/10, A61P 11/14

(54) **USE OF TRANS AMANTADINE DERIVATIVE OR SALT THEREOF**

(30) Priority: 17.03.2021 CN 202110284357
(71) Applicant: Chengdu Shibeikang Biomedical Technology Co., Ltd., Chengdu, Sichuan 611731 (CN)
(72) Inventor: MOU, Xia, Chengdu, Sichuan 611731 (CN); DONG, Bo, Chengdu, Sichuan 611731 (CN); ZHANG, Hai, Chengdu, Sichuan 611731 (CN); ZENG, Yanqun, Chengdu, Sichuan 611731 (CN); XU, Xia, Chengdu, Sichuan 611731 (CN); JIANG, Jie, Chengdu, Sichuan 611731 (CN)
(74) Representative: IP TRUST SERVICES
(86) International application number: PCT/CN2021/084409
(87) International publication number: WO 2022/193369

(57) **Abstract**

The present invention pertains to the field of pharmaceutical chemistry, and discloses the use of a trans-amantadine derivative having a structure represented by Formula I or a pharmaceutically acceptable salt thereof in the manufacture of a medicament for preventing or/and treating respiratory diseases. The trans-amantadine derivative and salt thereof according to the present invention have better efficacy than ambroxol hydrochloride and amantadine hydrochloride derivative racemate (mixture of isomers). From a mouse acute lung injury test and an ammonia-induced cough model in mice, it is shown to have excellent effects in treating lung injury and cough, which are were statistically superior to those of ambroxol hydrochloride and amantadine hydrochloride derivative racemate. The trans-amantadine ammonia derivative and its hydrochloride salt of the present invention can significantly increase the amount of sputum excreted by rats compared with ambroxol hydrochloride, and can significantly increase the excretion of phenol red in mice, with effects better than those of ambroxol hydrochloride and amantadine hydrochloride derivative racemate.

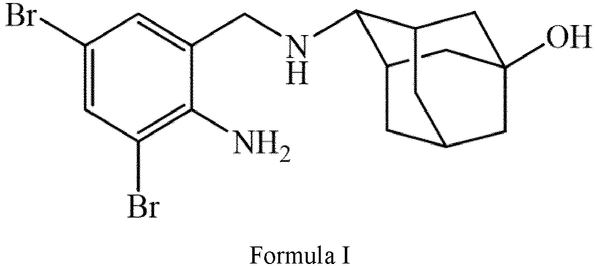

## Description

### Technical Field

The present invention relates to the pharmaceutical field, more particularly to the use of a trans-amantadine derivative or a pharmaceutically acceptable salt thereof.

### Background

Lung injury is injury to the lung parenchyma caused by different injury factors, and may manifest as lung laceration, lung contusion and lung blast injury. The most common is pulmonary contusion, with an incidence rate of 30% to 75% amongst blunt chest injuries and a mortality rate of 14% to 40%. Lung trauma, lung surgery, lung infection, pulmonary embolism and the like may also cause lung injury. The rate of lung injury continues to rise worldwide, and no specific drug is available, but analgesics and anti-infective drugs are mainly used. Therefore, there is an urgent clinical need for safe and effective drugs that can treat lung injury, especially drugs that treat acute lung injury.

Coughing is one of the biological defense responses to exhale phlegm and foreign substances in the airways, but if sustained and excessive, it can lead to decreased quality of life (QOL). Cough is common in clinical practice, especially chronic cough lasting for over 8 weeks, which accounts for more than 1/3 of respiratory outpatient visits and seriously affects patients' work and daily life. Increased cough sensitivity caused by airway neurogenic inflammation is an important mechanism. Questionnaire surveys in European and American countries show that up to 9-33% of people suffer from coughs of varying degrees; in Japan, the incidence of chronic cough varies according to reports, but is approximately 2% to 10%; in 2006, a survey of 1,087 college students in Guangzhou, China showed that the incidence rate of cough was 10.9%, of which the incidence rate of chronic cough was 3.3%. It is speculated that the incidence rate in the community population would be higher. There are currently no drug approved to treat chronic cough. Commonly used antitussive agents include codeine, dextromethorphan, etc., but central antitussive agents often cause side effects such as constipation and drowsiness. Therefore, there is an urgent clinical need for safe and effective drugs to treat cough, especially drugs to treat chronic cough.

Ambroxol is approved in many countries for acute and chronic lung diseases associated with abnormal sputum secretion and poor sputum function. As such, the main function of ambroxol is an expectorant effect, and its therapeutic effect on lung injury, especially acute lung injury, is poor. Further, Japan has also approved ambroxol for the treatment of cough.

Patent CN201910671508.7 discloses dibromobenzyl derivatives, stereoisomers or salts, and the preparation method and use thereof. The dibromobenzyl derivatives as disclosed are obtained by structural modification of the cyclohexane in ambroxol. The disclosed dibromobenzyl derivatives have better bioavailability and better expectorant effect than ambroxol hydrochloride. At present, there are no relevant reports on the use of trans-amantadine derivatives in the prevention and treatment of lung injury and cough, especially reports on acute lung injury and refractory cough.

### Summary

An object of the present invention is to provide the use of a trans-amantadine derivative represented by Formula I or a pharmaceutically acceptable salt thereof in the preparation of a medicament for preventing or/and treating respiratory diseases.

In order to achieve the above object, the technical solutions adopted by the present invention are as follows:
Use of the trans-amantadine derivatives having a structure represented by Formula I or a pharmaceutically acceptable salt thereof according to the present invention in the preparation of a medicament for preventing or/and treating respiratory diseases,

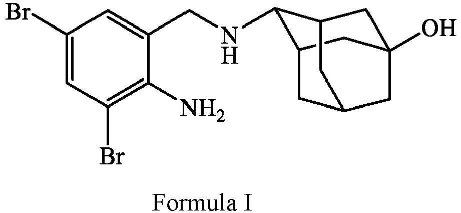

In certain embodiments of the present invention, comprised is the use in the manufacture of a medicament for preventing or/and treating lung injury.

Further, comprised is the use in the manufacture of a medicament for preventing or/and treating acute lung injury.

Further, comprised is the use in the manufacture of a medicament for preventing or/and treating acute respiratory distress syndrome caused by lung injury.

In certain embodiments of the present invention, comprised is the use in the manufacture of a medicament for preventing or/and treating cough.

Further, comprised is the use in the manufacture of a medicament for preventing or/and treating chronic cough or refractory cough.

In certain embodiments of the present invention, comprised is the use in the manufacture of a medicament as a preventive or/and therapeutic expectorant.

In some embodiments of the present invention, the pharmaceutically acceptable salt is formed from a trans-adamantane derivative with an acid.

The acid is a pharmaceutically acceptable inorganic acid or organic acid, wherein the inorganic acid is selected from hydrochloric acid, hydrosulfuric acid, hydrobromic acid, hydroiodic acid, sulfuric acid, nitric acid, boric acid, and phosphoric acid; and the organic acid is selected from formic acid, acetic acid , acetic anhydride, acetoacetic acid, trifluoroacetic acid, propionic acid, pyruvic acid, butyric acid, caproic acid, heptanoic acid, undecanoic acid, lauric acid, stearic acid, palmitic acid, oxalic acid, malonic acid, succinic acid, glutaric acid, adipic acid, maleic acid, fumaric acid, lactic acid, malic acid, citric acid, tartaric acid, metatartaric acid, ascorbic acid, gallic acid, benzoic acid, salicylic acid, cinnamic acid, naphthoic acid, embonic acid, nicotinic acid, orotic acid, phytic acid, methyl sulfate, dodecyl sulfate, methanesulfonic acid, trifluoromethanesulfonic acid, ethylene disulfonic acid, isethionic acid, 1,5-naphthalenedisulfonic acid, 2-naphthalenedisulfonic acid, camphorsulfonic acid, hydrogen sulfonic acid, glutamic acid, aspartic acid, gluconic acid, and glucuronic acid; preferably, the pharmaceutically acceptable acid is hydrochloric acid, hydrosulfuric acid, hydrobromic acid, sulfuric acid, phosphoric acid, acetic acid, lactic acid, malic acid, tartaric acid, or fumaric acid.

Preferably, the pharmaceutically acceptable salts include acetate, hydrosulfate, ascorbate, benzoate, benzenesulfonate, citrate, fumarate, hydrochloride, hydrobromide, maleate, methanesulfonate, nitrate, oxalate, phosphate, succinate, or sulfate.

Preferably, the pharmaceutically acceptable salt is selected from,
*trans*-4-[(2-amino-3,5-dibromobenzyl)amino]adamantan-1-ol hydrochloride,
*trans*-4-[(2-amino-3,5-dibromobenzyl)amino]adamantan-1-ol hydrosulfate, or
*trans*-4-[(2-amino-3,5-dibromobenzyl)amino]adamantan-1-ol hydrobromide.

Preferably, the pharmaceutically acceptable salt is a hydrochloride having a structure represented by Formula V,

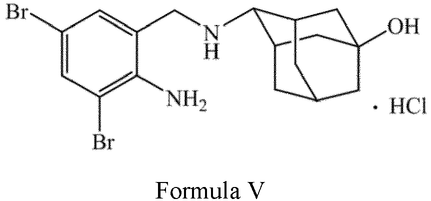

The preparation method of trans-amantadine derivatives having a structure represented by Formula I according to the present invention includes a reaction having two steps:
Step 1: using a compound of Formula III, a compound of Formula IV or a salt thereof as a starting material, preparing the compound of Formula II through a condensation reaction;
Step 2: reducing the compound of Formula II with a reducing agent to obtain the trans-amantadine derivative represented by Formula I;

The reaction scheme is as follows:

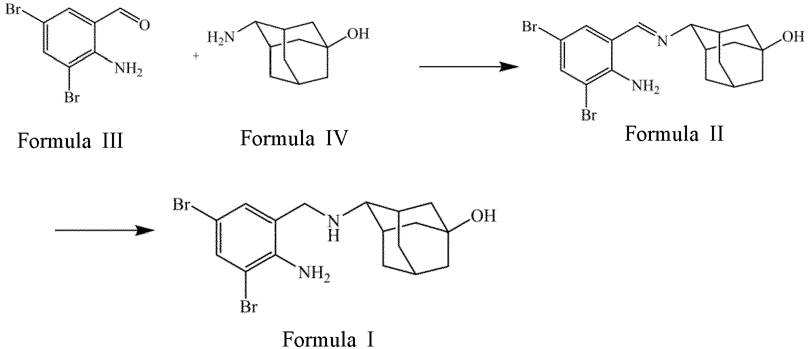

In some embodiments of the present invention, Step 3 is further included where the compound of Formula I is reacted with an acid to form a salt to prepare a salt of the compound of Formula I. The reaction scheme is as follows:

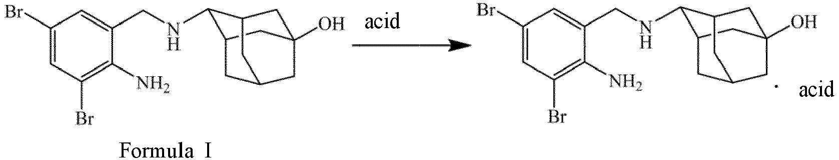

In some embodiments of the present invention, the reducing agent includes but is not limited to sodium borohydride or potassium borohydride.

In some embodiments of the present invention, the salt of the compound of Formula IV includes, but is not limited to, hydrochloride, sulfate, and hydrobromide.

The above Step 1 includes adding a reaction solvent, the compound of Formula III, the compound of Formula IV or salt thereof, an acid-capturer, and a desiccant into a reactor, and heating and stirring the reaction to prepare the compound of Formula II.

The compound of Formula III and the compound of Formula IV as starting materials are at a molar ratio of 1:0.8 to 1:2.0, preferably 1: 1 to 1:1.5.

The acid-capturer includes an inorganic base or/and an organic base; preferably, the inorganic base includes but is not limited to any one or more of sodium bicarbonate, potassium bicarbonate, sodium carbonate, potassium carbonate, cesium carbonate, sodium hydroxide, potassium hydroxide, lithium hydroxide or sodium hydride;
preferably, the organic base includes but is not limited to any one or more of sodium methoxide, sodium ethoxide, potassium tert-butoxide, triethylamine, diethylamine, diisopropylamine, and N,N-diisopropylethylamine.

The molar ratio of the compound of Formula III to the acid-capturer is 1:1 to 1:5.0, preferably 1:1 to 1:1.5.

The reaction solvent includes but is not limited to any one or more of ethanol, methanol, isopropanol, n-propanol, ethyl acetate, isopropyl acetate, N,N-dimethylformamide, N,N-dimethylacetamide, dimethyl sulfoxide, acetonitrile, and tetrahydrofuran.

Preferably, the mass-to-volume ratio of the compound of Formula III (2-amino-3,5-dibromobenzaldehyde) to the reaction solvent is 1:3 to 1:20, preferably 1:12 to 1:16, in which when a unit in mass is kg, the unit in volume is L.

The desiccant includes but is not limited to any one or more of magnesium sulfate, sodium sulfate, calcium sulfate, molecular sieves, and calcium chloride; preferably, the molar ratio of the compound of Formula III (2-amino-3,5-dibromobenzene formaldehyde) to the desiccant is 1:0.5 to 1:5, preferably 1:0.8 to 1:1.5.

The reaction temperature in Step 1 is 50°C to 120°C, preferably 70°C to 80°C.

Step 1 also includes a post-treatment step which includes filtering, concentrating, crystallizing, and drying the reaction solution to obtain the compound of Formula II.

When the compound of Formula II is purified by recrystallization, the solvent for recrystallization includes but is not limited to any one or more of methanol, ethanol, isopropyl alcohol, and n-propanol; the weight-to-volume ratio of the compound of Formula III to the recrystallization solvent is: 1:4 to 1:25, preferably 1:9 to 1:11; when the unit in weight is kg, the unit in volume is L.

In some embodiments of the present invention, the reaction solvent in Step 2 includes but is not limited to one or two of dichloromethane and methanol; the mass volume ratio of the compound of Formula II to the reaction solvent is 1:3 to 50, preferably 1:29, where for a unit in mass of kg, the unit in volume is L.

In Step 2, the reaction temperature is 0°C to 10°C, and the molar ratio of the compound of Formula II to the reducing agent is 1: 1.1 to 1:2.5.

In some embodiments of the present invention, the reaction solvent in Step 3 includes, but not limited to any one or more of ethanol, acetone, and methanol; the mass-to-volume ratio of the compound of Formula I to the reaction solvent is 1:3 to 70, preferably 1:12, or 1:14, or 1:29; where for a unit in mass of kg, the unit in volume is L.

The present invention further provides a pharmaceutical composition, including a trans-amantadine derivative represented by Formula I or a pharmaceutically acceptable salt thereof, and one or more pharmaceutically acceptable carrier(s).

The term "pharmaceutically acceptable carrier" refers to a diluent, adjuvant, excipient, or vehicle that is administered together with a therapeutic agent and is suitable, within the scope of reasonable medical judgment, for contact with tissues of humans and/or other animals without undue toxicity, irritation, allergic reactions, or other issues or complications commensurate with a reasonable benefit/risk ratio.

The present invention has the following advantageous effects over the prior art:
The present invention creatively structurally modifies the cyclohexane in ambroxol to obtain a trans-amantadine derivative represented by Formula I. It is surprisingly found in the present invention that the trans-amantadine derivative and its hydrochloride salt of the present invention have better efficacy than ambroxol hydrochloride and amantadine derivative racemate (mixture of isomers). From a mouse acute lung injury test and an ammonia-induced cough model in mice, it is shown to have excellent effects in treating lung injury and cough, which are better than those of ambroxol hydrochloride and amantadine hydrochloride derivative racemate, and are of statistical significance. The trans-amantadine ammonia derivative and its hydrochloride salt of the present invention can significantly increase the amount of sputum excreted by rats compared with ambroxol hydrochloride, and can significantly increase the excretion of phenol red in mice, with effects better than those of ambroxol hydrochloride and amantadine hydrochloride derivative racemate.

### Brief Description of the Drawings

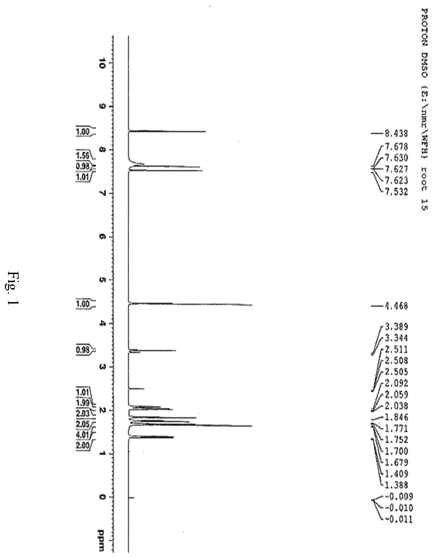

Fig. 1 is the ¹H-NMR spectrum (DMSO-d6) of the compound of Formula II, trans-4-[(2-amino-3,5-dibromophenylene)amino]-adamantane-1-ol;

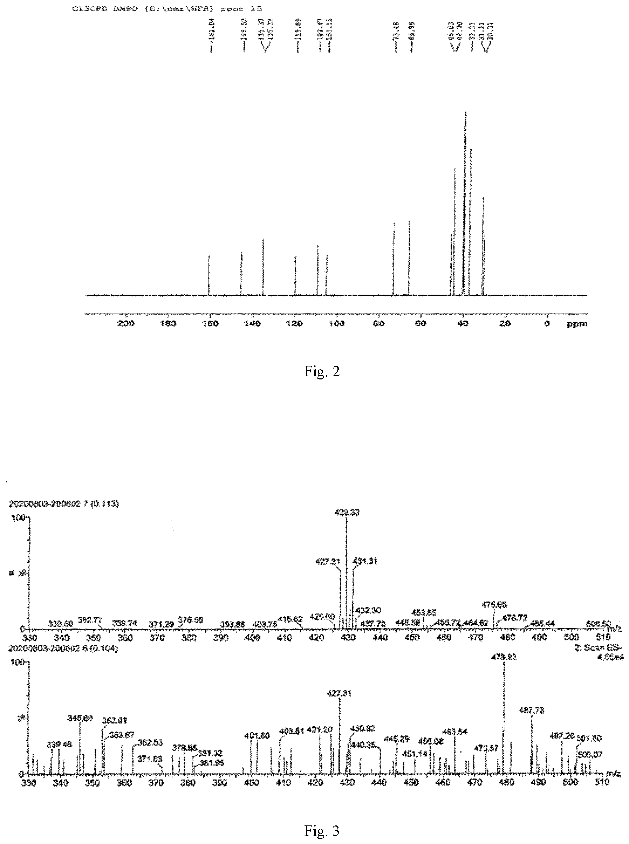

Fig. 2 is the ¹³C-NMR spectrum (DMSO-*d*6) of the compound of Formula II, trans-4-[(2-amino-3,5-dibromophenylene)amino]-adamantane-1-ol;
Fig. 3 is the mass spectrum of the compound of Formula II, trans-4-[(2-amino-3,5-dibromophenylene)amino]-adamantane-1-ol;

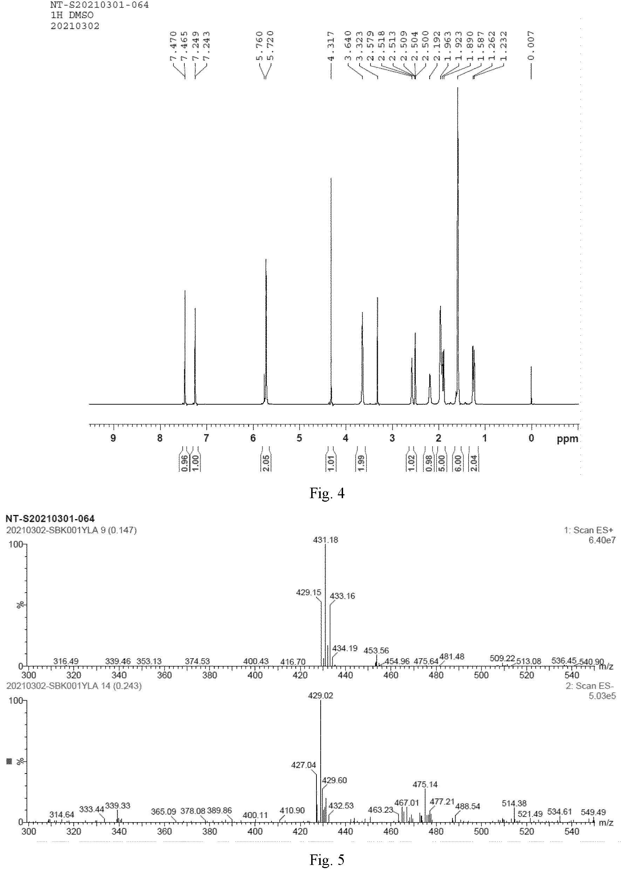

Fig. 4 is the ¹H-NMR spectrum (DMSO-*d*6) of the compound of Formula I, trans-4-[(2-amino-3,5-dibromobenzyl)amino]adamantan-1-ol;
Fig. 5 is the MS spectrum of the compound of Formula I, trans-4-[(2-amino-3,5-dibromobenzyl)amino]adamantan-1-ol;

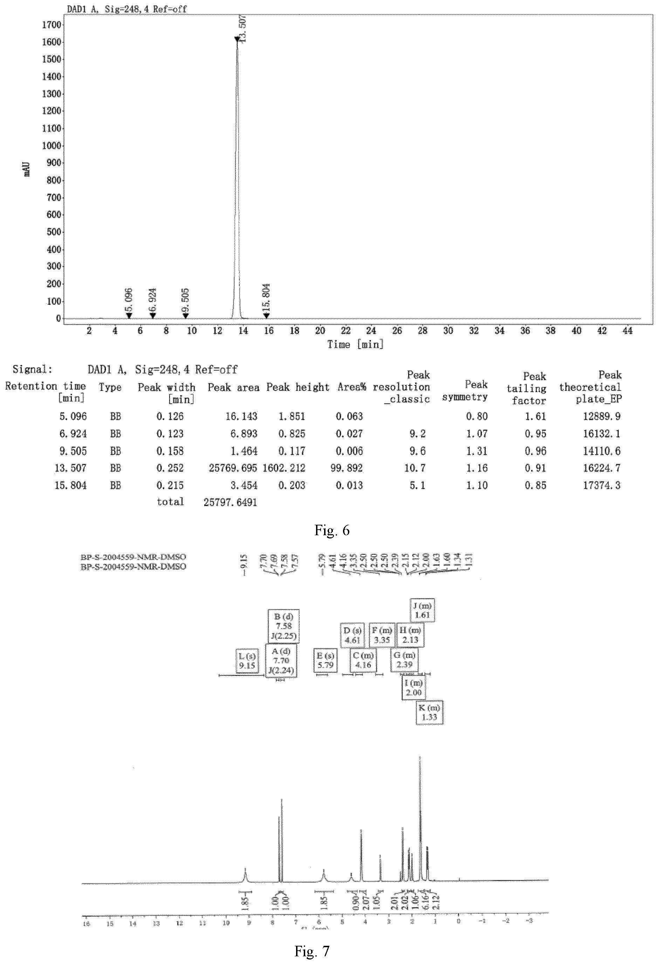

Fig. 6 is the HPLC spectrum of the compound of Formula V, trans-4-[(2-amino-3,5-dibromobenzyl)amino]adamantan-1-ol hydrochloride;
Fig. 7 is the ¹H-NMR spectrum (DMSO-*d*6) of the compound of Formula V, trans-4-[(2-amino-3,5-dibromobenzyl)amino]adamantan-1-ol hydrochloride;
Fig. 8 is a ¹³C-NMR spectrum (DMSO-*d*6) of the compound of Formula V, trans-4-[(2-amino-3,5-dibromobenzyl)amino]adamantan-1-ol hydrochloride;
Fig. 9 is the infrared absorption spectrum of the compound of Formula V, trans-4-[(2-amino-3,5-dibromobenzyl)amino]adamantan-1-ol hydrochloride;
Fig. 10 is a single crystal diffraction spectrum of the compound of Formula V, trans-4-[(2-amino-3,5-dibromobenzyl)amino]adamantan-1-ol hydrochloride.

### DETAILED DESCRIPTION OF THE PREFERRED EMBODIMENTS

For a better understanding of the object, technical solutions, and advantages of the embodiments of the present invention, the technical solutions in the embodiments of the present invention will be clearly and fully described below. If the specific conditions are not indicated in the examples, the conventional conditions or the conditions recommended by the manufacturer will be employed. For those without indication of the manufacturer of the reagents or instruments used, they are all conventional products that are commercially available.

In the following examples, the specific structure of the compound was confirmed by mass spectrometry (MS) and nuclear magnetic resonance (¹H NMR and ¹³C NMR). Among them, the nuclear magnetic resonance (¹H NMR and ¹³C NMR) shift (δ) is given in unit of parts per million (ppm); the nuclear magnetic resonance (¹H NMR) was measured with a Bruker AVANCE-400 nuclear magnetic instrument. All raw materials used in the examples of the present invention are commercially available.

Examples 1-17 disclose the preparation methods of the intermediates of the present invention.

### Example 1

This example discloses the preparation method of the intermediate trans-4-[(2-amino-3,5-dibromophenylene)amino]-adamantane-1-ol of the present invention, which specifically includes the steps as follows:

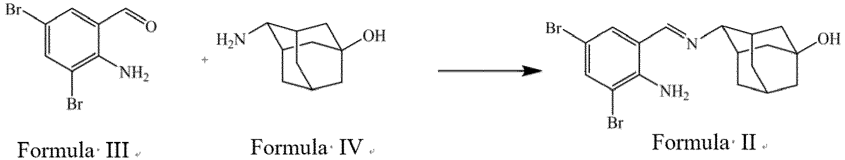

0.4 L of absolute ethanol was added to a reaction flask, and 27.9g of 2-amino-3,5-dibromobenzaldehyde (the compound of Formula III), 16.3g of trans-4-amino-1-hydroxyadamantane hydrochloride (the compound of formula IV), 15.9g anhydrous sodium carbonate, and 10.8g anhydrous magnesium sulfate were added thereto under stirring, and heated to 75°C and reacted for 5 hours or more. The reaction was completed as monitored by TLC, and the reaction solvent was evaporated under normal pressure. Then, 0.3 L of absolute ethanol was added to the residue, and the mixture was heated and recrystallized. After filtration and drying, 19.9g of trans-4-[2-amino-3,5-dibromophenylene)amino]-adamantan-1-ol was obtained as a light yellow powder. The actual yield was 58.0% and the purity was 99.1%.

¹H-NMR (600MHz, DMSO-*d*₆): 6ppm8.438(d, *J*=1.8Hz, 1H), 7.678(s, 2H), 7.63-7.623(t, *J*=2.5Hz, 1H), 7.532(t, *J*=2.2Hz, 1H), 4.468(s, 1H), 3.389-3.344(d, *J*=3.3Hz, 1H), 2.092(q, *J*=3.3Hz, 1H), 2.059-2.038(dd, *J*=12.8, 3.2Hz, 2H), 1.846(m, 2H), 1.771-1.752(d, *J*=11.8Hz, 2H), 1.700-1.679(dd, *J*=13.0, 3.0Hz, 4H), 1.409-1.388(d, *J*=12.3Hz, 2H), which is consistent with the structural formula.

¹³C-NMR(150MHz, DMSO-*d*₆): δppm161.04, 145.52, 135.37, 135.32, 119.89, 109.47, 105.15, 73.48, 65.99, 46.03, 44.70, 37.31, 31.11, 30.31, which is consistent with the structural formula.

The theoretical value M of MS molecular formula C₁₇H₂₀Br₂N₂O is 428.17. The molecular ion peak in the mass spectrum of m/z 429.33 is the [M+H]⁺ peak; the molecular ion peak in the mass spectrum of m/z 427.31 is the [M-H]⁻ peak, which is consistent with the structural formula.

### Example 2

This example discloses the preparation method of the intermediate trans-4-[(2-amino-3,5-dibromophenylene)amino]-adamantane-1-ol of the present invention, which specifically includes the steps as follows:
0.4 L of absolute ethanol was added to a reaction flask, and 27.9g of 2-amino-3,5-dibromobenzaldehyde (the compound of Formula III), 20.4g of trans-4-amino-1-hydroxyadamantane hydrochloride (the compound of formula IV), 15.9g anhydrous sodium carbonate, and 10.8g anhydrous magnesium sulfate were added thereto under stirring, and heated to 75°C and reacted for 5 hours or more. The reaction was completed as monitored by TLC, and the reaction solvent was evaporated under normal pressure. Then, 0.25 L of absolute ethanol was added to the residue, and the mixture was heated and recrystallized. After filtration and drying, 32.9g of trans-4-[2-amino-3,5-dibromophenylene)amino]-adamantan-1-ol was obtained as a light yellow powder. The actual yield was 76.8% and the purity was 98.9%.

### Example 3

This example discloses the preparation method of the intermediate trans-4-[(2-amino-3,5-dibromophenylene)amino]-adamantane-1-ol of the present invention, which specifically includes the steps as follows:
0.4 L of absolute ethanol was added to a reaction flask, and 27.9g of 2-amino-3,5-dibromobenzaldehyde (the compound of Formula III), 30.5g of trans-4-amino-1-hydroxyadamantane hydrochloride (the compound of formula IV), 15.9g anhydrous sodium carbonate, and 10.8g anhydrous magnesium sulfate were added thereto under stirring, and heated to 75°C and reacted for 5 hours or more. The reaction was completed as monitored by TLC, and the reaction solvent was evaporated under normal pressure. Then, 0.25 L of absolute ethanol was added to the residue, and the mixture was heated and recrystallized. After filtration and drying, 36.4g of trans-4-[2-amino-3,5-dibromophenylene)amino]-adamantan-1-ol was obtained as a light yellow powder. The actual yield was 85.0% and the purity was 98.6%.

### Example 4

This example discloses the preparation method of the intermediate trans-4-[(2-amino-3,5-dibromophenylene)amino]-adamantane-1-ol of the present invention, which specifically includes the steps as follows:
0.4 L of absolute ethanol was added to a reaction flask, and 27.9g of 2-amino-3,5-dibromobenzaldehyde (the compound of Formula III), 40.7g of trans-4-amino-1-hydroxyadamantane hydrochloride (the compound of formula IV), 15.9g anhydrous sodium carbonate, and 10.8g anhydrous magnesium sulfate were added thereto under stirring, and heated to 75°C and reacted for 5 hours or more. The reaction was completed as monitored by TLC, and the reaction solvent was evaporated under normal pressure. Then, 0.25 L of absolute ethanol was added to the residue, and the mixture was heated and recrystallized. After filtration and drying, 34.7g of trans-4-[2-amino-3,5-dibromophenylene)amino]-adamantan-1-ol was obtained as a light yellow powder. The actual yield was 81.0% and the purity was 99.7%.

### Example 5

This example discloses the preparation method of the intermediate trans-4-[(2-amino-3,5-dibromophenylene)amino]-adamantane-1-ol of the present invention, which specifically includes the steps as follows:
0.4 L of absolute ethanol was added to a reaction flask, and 27.9g of 2-amino-3,5-dibromobenzaldehyde (the compound of Formula III), 22.4g of trans-4-amino-1-hydroxyadamantane hydrochloride (the compound of formula IV), 15.9g anhydrous sodium carbonate, and 10.8g anhydrous magnesium sulfate were added thereto under stirring, and heated to 75°C and reacted for 5 hours or more. The reaction was completed as monitored by TLC, and the reaction solvent was evaporated under normal pressure. Then, 0.25 L of absolute ethanol was added to the residue, and the mixture was heated and recrystallized. After filtration and drying, 39.6g of trans-4-[2-amino-3,5-dibromophenylene)amino]-adamantan-1-ol was obtained as a light yellow powder. The actual yield was 92.5% and the purity was 99.6%.

### Example 6

This example discloses the preparation method of the intermediate trans-4-[(2-amino-3,5-dibromophenylene)amino]-adamantane-1-ol of the present invention, which specifically includes the steps as follows:
0.4 L of absolute ethanol was added to a reaction flask, and 27.9g of 2-amino-3,5-dibromobenzaldehyde (the compound of Formula III), 22.4g of trans-4-amino-1-hydroxyadamantane hydrochloride (the compound of formula IV), 20.7g anhydrous potassium carbonate, and 10.8g anhydrous magnesium sulfate were added thereto under stirring, and heated to 75°C and reacted for 5 hours or more. The reaction was completed as monitored by TLC, and the reaction solvent was evaporated under normal pressure. Then, 0.25 L of absolute ethanol was added to the residue, and the mixture was heated and recrystallized. After filtration and drying, 31.2g of trans-4-[2-amino-3,5-dibromophenylene)amino]-adamantan-1-ol was obtained as a light yellow powder. The actual yield was 72.9% and the purity was 98.1%.

### Example 7

This example discloses the preparation method of the intermediate trans-4-[(2-amino-3,5-dibromophenylene)amino]-adamantane-1-ol of the present invention, which specifically includes the steps as follows:
0.4 L of a mixed solvent of absolute ethanol and tetrahydrofuran (1:1) was added to a reaction flask, and 27.9g of 2-amino-3,5-dibromobenzaldehyde (the compound of Formula III), 24.4g of trans-4-amino-1-hydroxyadamantane hydrochloride (the compound of formula IV), 15.9g anhydrous sodium carbonate, and 10.8g anhydrous magnesium sulfate were added thereto under stirring, and heated to 75°C and reacted for 5 hours or more. The reaction was completed as monitored by TLC, and the reaction solvent was evaporated under normal pressure. Then, 0.25 L of absolute ethanol was added to the residue, and the mixture was heated and recrystallized. After filtration and drying, 30.1g of trans-4-[2-amino-3,5-dibromophenylene)amino]-adamantan-1-ol was obtained as a light yellow powder. The actual yield was 70.3% and the purity was 98.5%.

### Example 8

This example discloses the preparation method of the intermediate trans-4-[(2-amino-3,5-dibromophenylene)amino]-adamantane-1-ol of the present invention, which specifically includes the steps as follows:
0.4 L of tetrahydrofuran was added to a reaction flask, and 27.9g of 2-amino-3,5-dibromobenzaldehyde (the compound of Formula III), 24.4g of trans-4-amino-1-hydroxyadamantane hydrochloride (the compound of formula IV), 15.9g anhydrous sodium carbonate, and 10.8g anhydrous magnesium sulfate were added thereto under stirring, and heated to 75°C and reacted for 5 hours or more. The reaction was completed as monitored by TLC, and the reaction solvent was evaporated under normal pressure. Then, 0.3 L of absolute ethanol was added to the residue, and the mixture was heated and recrystallized. After filtration and drying, 27.8g of trans-4-[2-amino-3,5-dibromophenylene)amino]-adamantan-1-ol was obtained as a light yellow powder. The actual yield was 64.9% and the purity was 99.5%.

### Example 9

This example discloses the preparation method of the intermediate trans-4-[(2-amino-3,5-dibromophenylene)amino]-adamantane-1-ol of the present invention, which specifically includes the steps as follows:
0.4 L of absolute ethanol was added to a reaction flask, and 27.9g of 2-amino-3,5-dibromobenzaldehyde (the compound of Formula III), 22.4g of trans-4-amino-1-hydroxyadamantane hydrochloride (the compound of formula IV), 10.5g anhydrous sodium carbonate, and 7.1g anhydrous sodium sulfate were added thereto under stirring, and heated to 50°C and reacted for 5 hours or more. The reaction was completed as monitored by TLC, and the reaction solvent was evaporated under normal pressure. Then, 0.25 L of absolute methanol was added to the residue, and the mixture was heated and recrystallized. After filtration and drying, 30.5g of trans-4-[2-amino-3,5-dibromophenylene)amino]-adamantan-1-ol was obtained as a light yellow powder. The actual yield was 71.2% and the purity was 99.4%.

### Example 10

This example discloses the preparation method of the intermediate trans-4-[(2-amino-3,5-dibromophenylene)amino]-adamantane-1-ol of the present invention, which specifically includes the steps as follows:
0.15 L of DMF was added to a reaction flask, and 27.9g of 2-amino-3,5-dibromobenzaldehyde (the compound of Formula III), 24.4g of trans-4-amino-1-hydroxyadamantane hydrochloride (the compound of formula IV), 4.0g sodium hydroxide, and 9.6g anhydrous magnesium sulfate were added thereto under stirring, and heated to 90°C and reacted for 5 hours or more. The reaction was completed as monitored by TLC, and the reaction solvent was evaporated under normal pressure. Then, 0.3 L of absolute methanol was added to the residue, and the mixture was heated and recrystallized. After filtration and drying, 27.9g of trans-4-[2-amino-3,5-dibromophenylene)amino]-adamantan-1-ol was obtained as a light yellow powder. The actual yield was 65.2% and the purity was 98.5%.

### Example 11

This example discloses the preparation method of the intermediate trans-4-[(2-amino-3,5-dibromophenylene)amino]-adamantane-1-ol of the present invention, which specifically includes the steps as follows:
0.45 L of absolute ethanol was added to a reaction flask, and 27.9g of 2-amino-3,5-dibromobenzaldehyde (the compound of Formula III), 24.4g of trans-4-amino-1-hydroxyadamantane hydrochloride (the compound of formula IV), 5.6 potassium hydroxide, and 10.8g anhydrous magnesium sulfate were added thereto under stirring, and heated to 70°C and reacted for 5 hours or more. The reaction was completed as monitored by TLC, and the reaction solvent was evaporated under normal pressure. Then, 0.3 L of absolute ethanol was added to the residue, and the mixture was heated and recrystallized. After filtration and drying, 33.0g of trans-4-[2-amino-3,5-dibromophenylene)amino]-adamantan-1-ol was obtained as a light yellow powder. The actual yield was 77.1% and the purity was 97.5%.

### Example 12

This example discloses the preparation method of the intermediate trans-4-[(2-amino-3,5-dibromophenylene)amino]-adamantane-1-ol of the present invention, which specifically includes the steps as follows:
0.35 L of absolute ethanol was added to a reaction flask, and 27.9g of 2-amino-3,5-dibromobenzaldehyde (the compound of Formula III), 24.4g of trans-4-amino-1-hydroxyadamantane hydrochloride (the compound of formula IV), 15.9g anhydrous sodium carbonate, and 18.1g anhydrous magnesium sulfate were added thereto under stirring, and reacted under reflux for 5 hours or more. The reaction was completed as monitored by TLC, and the reaction solvent was evaporated under normal pressure. Then, 0.1 L of absolute methanol was added to the residue, and the mixture was heated and recrystallized. After filtration and drying, 36.8g of trans-4-[2-amino-3,5-dibromophenylene)amino]-adamantan-1-ol was obtained as a light yellow powder. The actual yield was 85.9% and the purity was 92.2%.

### Example 13

This example discloses the preparation method of the intermediate trans-4-[(2-amino-3,5-dibromophenylene)amino]-adamantane-1-ol of the present invention, which specifically includes the steps as follows:
0.55 L of isopropanol was added to a reaction flask, and 27.9g of 2-amino-3,5-dibromobenzaldehyde (the compound of Formula III), 24.4g of trans-4-amino-1-hydroxyadamantane hydrochloride (the compound of formula IV), 48.8g anhydrous cesium carbonate, and 24.0g anhydrous magnesium sulfate were added thereto under stirring, and heated to 75°C and reacted for 5 hours or more. The reaction was completed as monitored by TLC, and the reaction solvent was evaporated under normal pressure. Then, 0.4 L of isopropanol was added to the residue, and the mixture was heated and recrystallized. After filtration and drying, 35.0g of trans-4-[2-amino-3,5-dibromophenylene)amino]-adamantan-1-ol was obtained as a light yellow powder. The actual yield was 81.7% and the purity was 98.8%.

### Example 14

This example discloses the preparation method of the intermediate trans-4-[(2-amino-3,5-dibromophenylene)amino]-adamantane-1-ol of the present invention, which specifically includes the steps as follows:
0.1 L of MDSO was added to a reaction flask, and 27.9g of 2-amino-3,5-dibromobenzaldehyde (the compound of Formula III), 24.4g of trans-4-amino-1-hydroxyadamantane hydrochloride (the compound of formula IV), 12.6g anhydrous sodium bicarbonate, and 5.6g calcium chloride were added thereto under stirring, and heated to 120°C and reacted for 5 hours or more. The reaction was completed as monitored by TLC, and the reaction solvent was evaporated under normal pressure. Then, 0.4 L of anhydrous ethanol was added to the residue, and the mixture was heated and recrystallized. After filtration and drying, 32.1g of trans-4-[2-amino-3,5-dibromophenylene)amino]-adamantan-1-ol was obtained as a light yellow powder. The actual yield was 75.0% and the purity was 98.7%.

### Example 15

This example discloses the preparation method of the intermediate trans-4-[(2-amino-3,5-dibromophenylene)amino]-adamantane-1-ol of the present invention, which specifically includes the steps as follows:
0.2 L of anhydrous ethanol was added to a reaction flask, and 27.9g of 2-amino-3,5-dibromobenzaldehyde (the compound of Formula III), 24.4g of trans-4-amino-1-hydroxyadamantane hydrochloride (the compound of formula IV), 82 mL N,N-diisopropylethylamine, and 10.8g anhydrous calcium sulfate were added thereto under stirring, and heated to 75°C and reacted for 5 hours or more. The reaction was completed as monitored by TLC, and the reaction solvent was evaporated under normal pressure. Then, 0.7 L of n-propanol was added to the residue, and the mixture was heated and recrystallized. After filtration and drying, 26.1g of trans-4-[2-amino-3,5-dibromophenylene)amino]-adamantan-1-ol was obtained as a light yellow powder. The actual yield was 61.0% and the purity was 99.1%.

### Example 16

This example discloses the preparation method of the intermediate trans-4-[(2-amino-3,5-dibromophenylene)amino]-adamantane-1-ol of the present invention, which specifically includes the steps as follows:
15 L of anhydrous ethanol was added to a kettle, and 995.8g of 2-amino-3,5-dibromobenzaldehyde (the compound of Formula III), 800.0g of trans-4-amino-1-hydroxyadamantane hydrochloride (the compound of formula IV), 567.6g anhydrous sodium carbonate, and 400.0g anhydrous magnesium sulfate were added thereto under stirring, heated and maintained at 75±5°C, and reacted for 10 hours or more. The reaction was completed as monitored by TLC, and the reaction solvent was evaporated under normal pressure. Then, 2 L of anhydrous ethanol was added to the residue, and the mixture was heated and recrystallized. After filtration and drying, 1381.9g of trans-4-[2-amino-3,5-dibromophenylene)amino]-adamantan-1-ol was obtained as a light yellow powder. The actual yield was 90.0% and the purity was 99.6%.

Examples 17-20 disclose the preparation methods of trans-4-[(2-amino-3,5-dibromobenzyl)amino]adamantan-1-ol and its salt.

### Example 17

This example discloses the preparation of the compound of Formula I, trans-4-[(2-amino-3,5-dibromobenzyl)amino]adamantan-1-ol, and the reaction scheme is as follows:

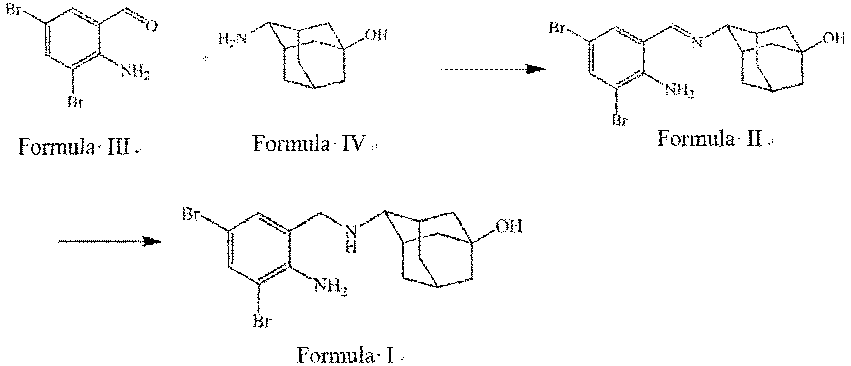

The preparation method is specifically as follows:

### Step 1: Preparation of trans-4-[(2-amino-3,5-dibromophenylene)amino]-adamantane-1-ol (Compound of Formula II)

20.4g of trans-4-amino-1-hydroxyadamantane hydrochloride (0.1 mol), 55.6g of 2-amino-3,5-dibromobenzaldehyde (0.36 mol) and 500 mL of absolute ethanol (EtOH) were added to a reaction flask, 10g of molecular sieve was further added, and refluxed for 10 hours. The reaction was complete as monitored by TLC, and the reaction solvent was evaporated under normal pressure. 250 mL of absolute ethanol was added to the residue, heated and recrystallized, and cooled and filtered to obtain 36.4 g of trans-4-[2-amino-3,5-dibromophenylene]amino]-adamantane-1-ol. The yield was 85% and the purity was 98.5%.

### Step 2: Preparation of trans-4-[(2-amino-3,5-dibromobenzyl)amino]adamantan-1-ol (Compound of Formula I)

34.3g (0.08 mol) of the intermediate compound of Formula II, 500 mL of methylene chloride, and 500 mL of anhydrous methanol were added into a reaction flask, dissolved under stirring, and 3.71g (0.098 mol) of sodium borohydride was then added. After the addition was complete, a reaction was carried out while maintaining at 5°C for 10 hours or more. The reaction was complete as monitored by TLC. After reaction the solvent was removed by concentration under reduced pressure, 100 mL of drinking water and 300 mL of methylene chloride were added to the residue, dissolved under stirring, and then separated, dried over anhydrous sodium sulfate, filtered, and rotary dried to obtain 31.36 g of the compound of Formula I, with a yield of 91% and a purity of 98.9%.

¹HNMR(400 MHz, DMSO-*d*₆): δ7.47 (d, *J*=2.00, 1H), 7.24 (d, *J*=2.40, 1H), 5.76-5.72 (m, 2H), 4. 32 (s, 1H), 3.64 (m, 2H), 2.52-2.50(m, 1H), 2.19 (m, 1H), 1.89-1.96 (m, 5H), 1.58 (m, 6H), 1.23- 1.26(m, 2H).

MS m/z (ES): 431.18 [M+H]⁺, 429.02 [M-H]⁻.

### Example 18

This example discloses the preparation of trans-4-[(2-amino-3,5-dibromobenzyl)amino]adamantan-1-ol hydrochloride (compound of Formula V). The reaction scheme is as follows:

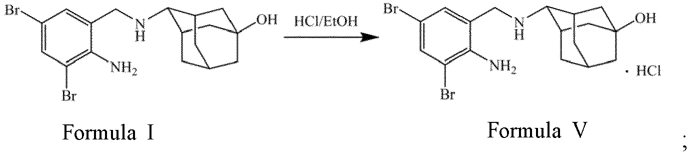

The preparation method is specifically as follows: 21.50g (0.05 mol) of the compound of Formula I prepared according to the method of Example 17 and 300 mL of absolute ethanol were added in a reaction flask, and heat to 75°C until the solid dissolved. 40 mL of a 15% hydrogen chloride (ethanol) solution was added dropwise to the substrate. After the addition was completed within 0.5 h, the heating was discontinued and it was allowed to cool naturally for crystal precipitation for about 3 h. Upon filtration, 19.8 g of trans-4-[(2-amino-3,5-dibromobenzyl)amino]adamantan-1-ol hydrochloride (Compound of Formula V) was obtained.

The HPLC purity was 99.89%, and the yield was 85%.

¹H NMR (400 MHz, DMSO-*d*₆): δ9.15 (s, 2H), 7.70 (d, *J*=2.24, 1H), 7.58 (d, *J*=2.25, 1H), 5.79 (s, 2H), 4. 61 (s, 1H), 4.16 (m, 2H), 3.35(s, 1H), 2.39 (m, 2H), 2.12-2.15 (m, 2H), 2.0 (m, 1H), 1.60-1.64(m, 6H), 1.31-1.34 (m, 2H).

¹³C NMR (400 MHz, DMSO-*d*₆): δ28.64, 28.64, 28.64, 28.80, 30.43, 30.43, 43.87, 44.91, 44.96, 61.71, 65.33, 106.33, 109.72, 119.01, 134.25, 134.46, 144.14.

Elemental analysis, measured values: 43.73% (C), 4.87% (H), 6.14% (N); theoretical values: 43.76% (C), 4.97% (H), 6.0% (N).

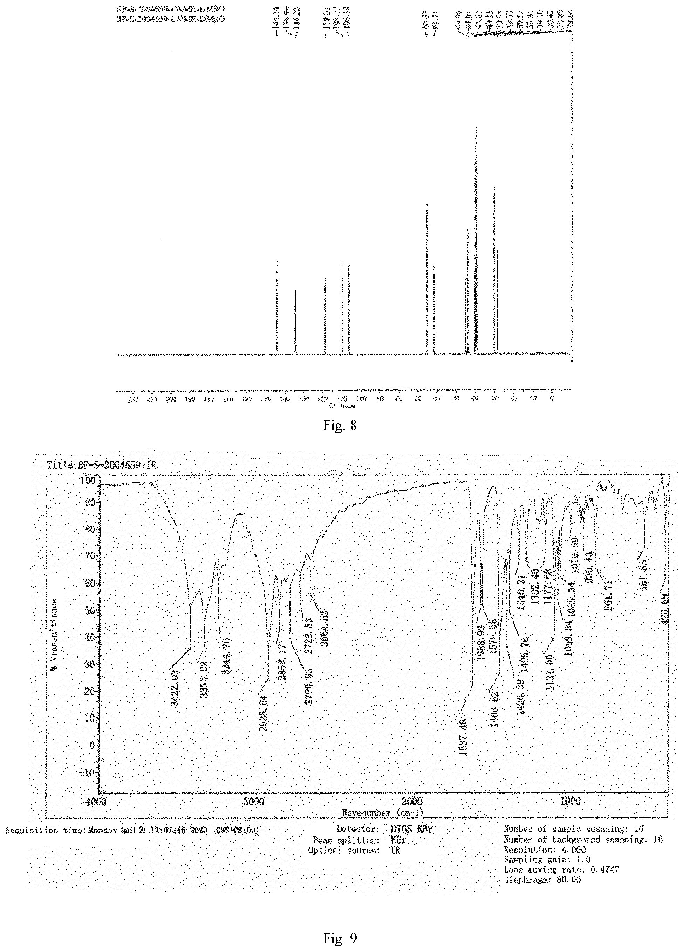

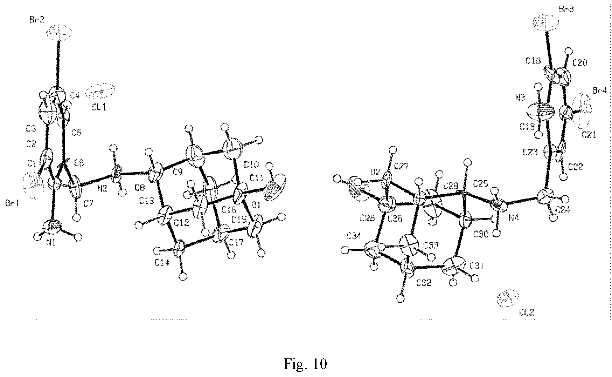

Further provided in the present application is the infrared absorption spectrum of the compound of Formula V for confirmation of its structure (as shown in Fig. 9). Moreover, the compound of Formula V of the invention was further subj ected to single crystal growth in the present application, and a single crystal refraction experiment was conducted (as shown in Fig. 10), which further confirms that the 4-[(2-amino-3,5-dibromobenzyl)amino]adamantane-1-ol of the present invention has a trans structure, and it was verified that the *trans* 4-[(2-amino-3,5-dibromobenzyl)amino]adamantane-1-ol of the present invention and HCl were in a ratio of 1:1 when they form a salt.

### Example 19

This example discloses the preparation of trans-4-[(2-amino-3,5-dibromobenzyl)amino]adamantan-1-ol hydrosulfate. The preparation method is specifically as follows:
4.3g (0.01 mol) of the compound of Formula I prepared according to the method of Example 17 and 50 mL of acetone were added into a reaction flask, cooled to about 10°C, and a solution of concentrated sulfuric acid in acetone (2 mL of concentrated sulfuric acid dissolved in 10 mL acetone) was added dropwise under stirring. The temperature was kept at 10°C while the stirring was continued for 2 hours, and then it was filtered to obtain 4.6g of trans-4-[(2-amino-3,5-dibromobenzyl)amino]adamantan-1-ol hydrosulfate.

The HPLC purity was 99.89%, and the yield was 87%.

¹HNMR(400 MHz, DMSO-*d*₆): δ9.10 (s, 2H), 7.69 (d, *J*=2.24, 1H), 7.57 (d, *J*=2.25, 1H), 5.78 (s, 2H), 4. 61 (s, 1H), 4.16 (m, 2H), 3.35(s, 1H), 2.39 (m, 2H), 2.12-2.15 (m, 2H), 2.0 (m, 1H), 1.60-1.64(m, 6H), 1.31-1.34 (m, 2H).

Elemental analysis, measured values: 38.79% (C), 4.35% (H), 5.37% (N); theoretical values: 38.73% (C), 4.40% (H), 5.31% (N).

### Example 20

This example discloses the preparation of trans-4-[(2-amino-3,5-dibromobenzyl)amino]adamantan-1-ol hydrobromide. The preparation method is specifically as follows:
4.3g (0.01 mol) of the compound of Formula I prepared according to the method of Example 17 and 300 mL of absolute ethanol were added into a reaction flask, and heated to 75°C until the solid was dissolved. 4 mL of 40% hydrogen bromide solution was added dropwise to the substrate. After the addition was complete within 0.5 h, the heating was discontinued and it was allowed to cool naturally for crystal precipitation for about 3 h. Upon filtration, 4.4 g of trans-4-[(2-amino-3,5-dibromobenzyl)amino]adamantan-1-ol hydrobromide was obtained.

The HPLC purity was 99.89%, and the yield was 86%.

¹HNMR(400 MHz, DMSO-*d*₆): δ9.12 (s, 2H), 7.70 (d, *J*=2.24, 1H), 7.58 (d,*J*=2.25, 1H), 5.79 (s, 2H), 4. 61 (s, 1H), 4.16 (m, 2H), 3.35(s, 1H), 2.39 (m, 2H), 2.12-2.15 (m, 2H), 2.0 (m, 1H), 1.60-1.64(m, 6H), 1.31-1.33 (m, 2H).

Elemental analysis, measured values: 39.99% (C), 4.43% (H), 5.57% (N); theoretical values: 39.95% (C), 4.54% (H), 5.48% (N).

### Comparative Example 1

### Preparation of 4-[(2-amino-3,5-dibromobenzyl)amino]adamantan-1-ol hydrochloride (racemic)

4-[(2-Amino-3,5-dibromobenzyl)amino]adamantan-1-ol hydrochloride (*racemic*) was synthesized according to the preparation method disclosed in Example 13 of patent CN201910671508.7.

In order to verify the performance and use of the compounds of the present invention, the efficacy of each of the compounds obtained in the above examples is demonstrated by the following Experiment Examples of the present invention as follows:

### Experiment Example 1

This Experiment Example discloses the acute lung injury tests in mouse of the compound of formula I and the compound of formula V.

KM mice, weighing 18-22g, with all 66 being males, were used and randomly grouped into a normal control group, a model control group, a Formula I compound group (40 mg/kg, in terms of free base), a Formula V compound group (40 mg/kg, in terms of free base), a Comparative Example group (40 mg/kg, in terms of free base), and an ambroxol hydrochloride group (40 mg/kg, in terms of free base), 11 animals in each group, and each group was administered intravenously (dissolved in normal saline by adjusting pH or directly dissolved in normal saline) once a day for 4 times, while the model control group and the normal control group were given an equivalent amount of normal saline. 30 minutes after the last administration, except for the normal control group, all other mice were administered 300 mg/kg oleic acid intravenously (prepared into the desired concentration with sterile saline containing 0.1% fetal calf serum immediately before use), and the vehicle control group was given an equivalent volume of 0.1% BSA saline. The mice were sacrificed 4 hours after the last administration, and the right lobes of the lungs were removed and fixed in a 10% formaldehyde solution. After dehydration, embedding in paraffin, dissection, and staining with HE, they were observed under a microscope for pathological changes of the lung tissues. The criteria for histological grading is shown in Table 1, and the results are shown in Table 2. Here, the medicament used for the Comparative Example group was the 4-[(2-amino-3,5-dibromobenzyl)amino]adamantan-1-ol hydrochloride (*racemic*) prepared in Comparative Example 1.

**Table 1. Criteria for histological grading of lung injury**

| Grading indicator | Grading criteria | Score |
|---|---|---|
| Alveolar wall thickening | No pathological change | 0 |
| | Occasional | 1 |
| | Mild | 2 |
| | Moderate | 3 |
| | Severe | 4 |
| Inflammatory cell infiltration | No pathological change | 0 |
| | Occasional | 1 |
| | Mild | 2 |
| | Moderate | 3 |
| | Severe | 4 |
| Lung tissue necrosis | No pathological change | 0 |
| | Occasional | 1 |
| | Mild | 2 |
| | Moderate | 3 |
| | Severe | 4 |

The above indicators are multiplied by different weighting numbers depending on the significance of the pathology (alveolar wall thickening × 1, inflammatory cell infiltration × 1, lung tissue necrosis × 3), and finally added up to obtain an overall lung pathology score.

**Table 2. Test results of lung pathologies of each group**

| Pathology | Level | Group | | | | | |
|---|---|---|---|---|---|---|---|
| | | Normal control | Model control | Ambroxol hydrochloride group | Formula I compound group | Comparative Example group | Formula V compound group |
| Alveolar wall thickening | 0 | 3 | 0 | 2 | 1 | 1 | 1 |
| | 1 | 4 | 3 | 4 | 7 | 2 | 7 |
| | 2 | 4 | 5 | 4 | 3 | 8 | 3 |
| | 3 | 0 | 3 | 1 | 1 | 0 | 0 |
| | 4 | 0 | 0 | 0 | 0 | 0 | 0 |
| Inflammatory cell infiltration | 0 | 4 | 0 | 0 | 3 | 1 | 2 |
| | 1 | 5 | 2 | 4 | 7 | 6 | 7 |
| | 2 | 2 | 9 | 7 | 1 | 4 | 2 |
| | 3 | 0 | 0 | 0 | 0 | 0 | 0 |
| | 4 | 0 | 0 | 0 | 0 | 0 | 0 |
| Lung tissue necrosis | 0 | 11 | 3 | 6 | 11 | 8 | 11 |
| | 1 | 0 | 4 | 4 | 0 | 2 | 0 |
| | 2 | 0 | 3 | 0 | 0 | 1 | 0 |
| | 3 | 0 | 1 | 1 | 0 | 0 | 0 |
| | 4 | 0 | 0 | 0 | 0 | 0 | 0 |
| Overall lung pathology score | | 21** | 81 | 54 | 23^{**▲} | 44* | 24^{**▲} |

| | | | | | | | |
|---|---|---|---|---|---|---|---|
| Note: Compared with the model control group: *P<0.05, **P<0.01; compared with the ambroxol hydrochloride group: AP<0.05. | | | | | | | |

As can be seen from Table 2 above: compared with the model control group, the compound of Formula I, the compound of Formula V, and the compound of the Comparative Example have a definite protective effect against acute lung injury in mice induced by oleic acid (P<0.05 or P<0.01). The overall pathology scores in the Formula I compound group and the Formula V compound group were significantly reduced compared with the ambroxol hydrochloride group, with a statistical difference (P<0.05), indicating that the compound of Formula I and compound of Formula V have a significantly better protective effect on the lungs than ambroxol hydrochloride, with the effect thereof also better than that of the compound of the Comparative Example.

Above all, the compound of Formula I and compound of Formula V have a protective effect against acute lung injury in mice induced by oleic acid, and their effects are better than that of ambroxol hydrochloride and the compound of the Comparative Example.

### Experiment Example 2

This Experiment Example discloses the effect test of the compound of formula I and the compound of formula V on the cough response of mice induced by ammonia water.

50 KM mice, weighing 18-22g, of both sexes were used and randomly divided into 5 groups, a model control group, a Formula I compound group (15 mg/kg, in terms of free base), a Formula V compound group (15 mg/kg, in terms of free base), a Comparative Example group (15 mg/kg, in terms of free base), and an ambroxol hydrochloride group (15 mg/kg, in terms of free base), 10 animals in each group, and were administered intravenously (dissolved in normal saline by adjusting pH or directly dissolved in normal saline). 30 minutes after the administration, the mice were put in an inverted beaker with a volume of 500ml and a cotton ball inside. Timing was started once 0.2ml of concentrated ammonia/mouse was added onto the cotton ball. The number of coughs of each animal within 3 minutes was observed and recorded. The results were subjected to the t' test, and the results are shown in Table 3. Here, the medicament used for the Comparative Example group was the 4-[(2-amino-3,5-dibromobenzyl)amino]adamantan-1-ol hydrochloride (*racemic*) prepared in Comparative Example 1.

**Table 3. Effect on ammonia-induced coughing response in mice (x ± s, n=10)**

| Group | Dosage (mg/kg) | Number of coughs (times) |
|---|---|---|
| Model control group | - | 41.7±8.76 |
| Formula I compound group | 15 | 19.2±5.98^{**▲▲} |
| Formula V compound group | 15 | 19.5±6.50^{**▲▲} |
| Comparative Example group | 15 | 25.7±6.40^{**▲} |
| Ambroxol hydrochloride group | 15 | 32.7±8.55^{*} |

| | | |
|---|---|---|
| Note: Compared with the model control group: *P<0.05, **P<0.01; compared with the ambroxol hydrochloride group: ▲P<0.05, ▲▲P<0.01. | | |

As can be seen from Table 3: compared with the model control group, the compound of formula I, the compound of formula V, the compound of the Comparative Example and ambroxol hydrochloride can significantly reduce the number of coughs (P<0.05 or P<0.01). The compound of formula I and the compound of formula V reduce the number of coughs compared with the ambroxol hydrochloride group with a statistical difference (P<0.01); and the compound of formula I and the compound of formula V reduce the number of coughs compared with the Comparative Example group with a statistical difference (P < 0.05), indicating that the antitussive effects of the compound of Formula I and the compound of Formula V are significantly better than that of ambroxol hydrochloride and the compound of the Comparative Example.

### Experiment Example 3

This Experiment Example discloses the rat capillary sputum excretion test of the compound of Formula I and the compound of Formula V.

180-220g SD rats of both sexes were used and divided into random groups, with 10 rats in each group, namely a vehicle control group, a Formula I compound group (15 mg/kg, in terms of free base), a Formula V compound group (15 mg/kg, in terms of free base), a Comparative Example group (15 mg/kg, in terms of free base) and ambroxol hydrochloride (15 mg/kg, in terms of free base). The rats were fasted and restrained from water for 12 hours before testing the amount of sputum excretion, and administered the respective medicaments via the tail vein (dissolved in normal saline by adjusting pH or directly dissolved in normal saline). 30 minutes after the administration, they were anesthetized by intraperitoneally injecting 1g/kg of a urethane solution in saline, fixed in supine position, and the trachea was separated after cutting through neck skin. A small hole was pierced by using a syringe needle between the two cartilage rings on the lower edge of the thyroid cartilage, and a capillary tube (with an inner diameter of 0.8mm, and a length of 10cm) was inserted centripetally into the trachea. Sputum was collected while adjusting the angle between the capillary tube and the trachea, and the total number of the length of liquids in the capillary tube (mm) was used to evaluate the expectoration effect. The amount of expectoration of the rats within 90 minutes was recorded. The amounts of expectoration in each group were statistically analyzed, and the results are shown in Table 4. Here, the medicament used for the Comparative Example group was the 4-[(2-amino-3,5-dibromobenzyl)amino]adamantan-1-ol hydrochloride (*racemic*) prepared in Comparative Example 1.

**Table 4. Results of the effect on the expectoration amount by rat capillary tube expectoration**

| **Group** | **Tested substance** | **Dosage (mg/kg)** | **Number of animals** | **Expectoration amount in 90min (mm)** |
|---|---|---|---|---|
| Vehicle control group | Saline | - | 10 | 22.7±4.41 |
| Formula I compound group | Compound of Formula I | 15 | 10 | 45.9±6.79^{*▲} |
| Formula V compound group | Compound of Formula V | 15 | 10 | 45.1±5.39^{*▲} |
| Comparative Example group | Compound of Comparative Example | 15 | 10 | 39.9±5.43^{*} |
| Ambroxol hydrochloride group | Ambroxol hydrochloride | 15 | 10 | 30.1±4.95^{*} |

| | | | | |
|---|---|---|---|---|
| Note: Compared with the control group: *P<0.05; compared with the control group, AP<0.05. | | | | |

As can be seen from Table 4: compared with the blank control group, all of the test substances can significantly increase the amount of expectoration in rats, with statistical differences (P<0.05); compared with the ambroxol hydrochloride group, both the compounds of Formula I, the compound of Formula V and the compound of the preparation Comparative Example can significantly increase the amount of expectoration in rats (P<0.05), which is significantly better than ambroxol hydrochloride; the compound of Formula I and the compound of Formula V can also significantly increase the amount of expectoration in rats compared with compound of the Comparative Example with a significant difference (P<0.05).

In summary, the compound of Formula I and the compound of Formula V can significantly increase the amount of expectoration in rats, and their effects are better than that of ambroxol hydrochloride and the compound of the Comparative Example.

### Experiment Example 4

This Experiment Example discloses the phenol red expectoration test in mice of the compound of Formula I and the compound of Formula V.

Experimental method: 60 mice of both sexes were used and randomly divided into a control (normal saline) group, a Formula I compound group (30 mg/kg, in terms of free base), a Formula V compound group (30 mg/kg, in terms of free base), a Comparative Example group (30 mg/kg, in terms of free base) and ambroxol hydrochloride (30 mg/kg, in terms of free base), 12 rats in each group; the respective medicaments were administered via the tail vein (dissolved in normal saline by adjusting pH or directly dissolved in normal saline) 2 times in a row. 15 minutes after the last administration to the mice, a 5% phenol red solution in normal saline (0.5g/kg) was subcutaneously injected. After 30 minutes, the mice were sacrificed and a section of the trachea was cut from below the thyroid cartilage to the trachea branch. The section was placed in a test tube containing 1 mL of normal saline, shaken and soaked for 30 minutes, and then centrifuged for 10 minutes (3000 r/min). The supernatant was transferred to another test tube, 0.1 mL of 1M NaOH solution was added, and shaken uniformly for a colorimetric measurement at 546nm. The effects of each group on the tracheal phenol red secretion in mice were statistically compared, and the experimental results obtained are shown in Table 5 below. Here, the medicament used for the Comparative Example group was the 4-[(2-amino-3,5-dibromobenzyl)amino]adamantan-1-ol hydrochloride (*racemic*) prepared in Comparative Example 1.

**Table 5. Effects on tracheal phenol red secretion in mice (x ± s)**

| Group | Tested substance | Dosage (mg/kg) | Number of animals (count) | Phenol red secretion (OD number) |
|---|---|---|---|---|
| Control group | Saline | - | 12 | 0.095±0.022 |
| Formula I compound group | Compound of Formula I | 30 | 12 | 0.279±0.063^{*▲} |
| Formula V compound group | Compound of Formula V | 30 | 12 | 0.278±0.054^{*▲} |
| Comparative Example group | Compound of Comparative Example | 30 | 12 | 0.223±0.058^{*} |
| Ambroxol hydrochloride group | Ambroxol hydrochloride | 30 | 12 | 0.135±0.023^{*} |

| | | | | |
|---|---|---|---|---|
| Note: Compared with the control group: *P<0.05; Compared with the control group, AP<0.05. | | | | |

As can be seen from Table 5 above: compared with the control group (normal saline), the test substances can all significantly increase the phenol red secretion of mice, with a statistical difference (P<0.05); compared with the ambroxol hydrochloride group, both the compound of Formula I, the compound of Formula V and the compound of the Comparative Example can significantly increase the excretion of phenol red (P<0.05), which is significantly better than ambroxol hydrochloride; the compound of Formula I, the compound of Formula V can also increase the excretion of phenol red significantly better than the Comparative Example group, with a significant difference (P<0.05).

In summary, the compound of Formula I and the compound of Formula V can significantly increase the phenol red excretion, and the effect thereof is better than ambroxol hydrochloride and the compound of the Comparative Example.

In summary according to the above, the compound of Formula I and the compound of Formula V have an antitussive effect on the ammonia-induced cough model in mice, with the effect better than that of ambroxol hydrochloride and the compound of the Comparative Example; the compound of Formula I and the compound of Formula V can significantly increase the phenol red excretion in mice, with the effect better than that of ambroxol hydrochloride and the compound of the Comparative Example.

The examples described above are only preferred embodiments of the present invention and should not be construed as a limitation to the scope of protection of the present invention. Any changes or modification without substantive significance made within the main idea and spirit of the present invention solve the same technical problems as the present invention does, and should be encompassed within the scope of protection of the present invention.

## Claims

1. Use of a trans-amantadine derivative having a structure represented by Formula I or a pharmaceutically acceptable salt thereof in the manufacture of a medicament for preventing or/and treating respiratory diseases,

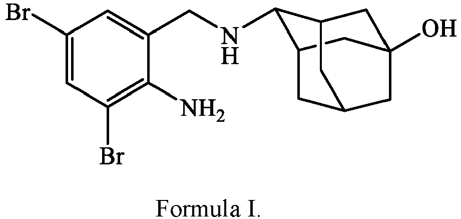

2. The use according to claim 1, **characterized in** including a use in the manufacture of a medicament for preventing or/and treating lung injury.

3. The use according to claim 2, **characterized in** including a use in the manufacture of a medicament for preventing or/and treating acute lung injury.

4. The use according to claim 2, **characterized in** including a use in the manufacture of a medicament for preventing or/and treating acute respiratory distress syndrome caused by lung injury.

5. The use according to claim 1, **characterized in** including a use in the manufacture of a medicament for preventing or/and treating cough.

6. The use according to claim 5, **characterized in** including a use in the manufacture of a medicament for preventing or/and treating chronic cough or refractory cough.

7. The use according to claim 1, **characterized in** including a use in the manufacture of a preventive or/and therapeutic expectorant.

8. The use according to any one of claims 1 to 7, **characterized in that** the pharmaceutically acceptable salt is formed from the trans-adamantane derivative with an acid.

9. The use according to claim 5, wherein the pharmaceutically acceptable salt includes acetate, hydrosulfate, ascorbate, benzoate, benzenesulfonate, citrate, fumarate, hydrochloride, hydrobromide, maleate, methanesulfonate, nitrate, oxalate, phosphate, succinate, or sulfate.

10. The use according to claim 9, **characterized in that** the pharmaceutically acceptable salt is selected from:
*trans*-4-[(2-amino-3,5-dibromobenzyl)amino]adamantan-1-ol hydrochloride, *trans*-4-[(2-amino-3,5-dibromobenzyl)amino]adamantan-1-ol hydrosulfate, and
*trans*-4-[(2-amino-3,5-dibromobenzyl)amino]adamantan-1-ol hydrobromide;
preferably, a hydrochloride having a structure represented by Formula V,
